# EUROPEAN PATENT SPECIFICATION

(11) **EP 2 047 854 B1**
(45) Date of publication and mention of the grant of the patent: **27.02.2013**
(21) Application number: 08164211.8
(22) Date of filing: 12.09.2008
(51) Int. Cl.: A61K 33/26, A61K 31/047, A61K 31/351, A61K 31/54, A61P 3/00, A61P 7/06

(54) **Pharmaceutical formulations containing ferrous bisglycinate chelate in combination with sweeteners (acesulfam K, sucralose, sorbitol) for improved palatability**
Pharmazeutische Formulierungen, die Eisen(II)-bisglycinat-Chelat in Kombination mit Süssstoffen (Acesulfam K, Sucralose, Sorbitol) enthalten für ein verbessertes Geschmacksempfinden
Formulations pharmaceutique contenant un chélate de bisglycinate ferreux en combinaison avec des edulcorants (acesulfam K, sucralose, sorbitol) pour donner un goût amélioré

(30) Priority: 12.10.2007 IT MI20071979
(43) Date of publication of application: 15.04.2009
(73) Proprietor: Baldacci, Massimo, 56125 Pisa (IT)
(72) Inventor: Baldacci, Massimo, 56125 Pisa (IT)
(74) Representative: Pistolesi, Roberto

(56) References cited:
- WO-A-2005/089779
- WO-A-2007/011018
- WO-A-2007/076857
- WO-A-2008/042218
- WO-A-2008/100767
- US-A- 5 504 055
- US-A1- 2003 190 355
- US-A1- 2004 082 502
- US-A1- 2005 196 503
- US-A1- 2006 134 227
- US-A1- 2007 148 103
- MIGLIORANZA LÚCIA H S ET AL: "Effect of long-term fortification of whey drink with ferrous bisglycinate on anemia prevalence in children and adolescents from deprived areas in Londrina, Paraná, Brazil." NUTRITION (BURBANK, LOS ANGELES COUNTY, CALIF.) MAY 2003, vol. 19, no. 5, May 2003 (2003-05), pages 419-421, XP002501198 ISSN: 0899-9007
- CROSS H L ET AL: "Carbohydrate/iron interactions. Effect of carbohydrate on iron recognition taste threshold values." LEBENSMITTEL-WISSENSCHAFT UND -TECHNOLOGIE 1984 DEP. OF FOOD TECH., UNIV. OF READING, WHITEKNIGHTS, READING RG1 5AQ, UK, vol. 17, no. 1, 1984, page 11, XP009107776

## Description

Ferrous bisglycinate chelate has the following structure formula

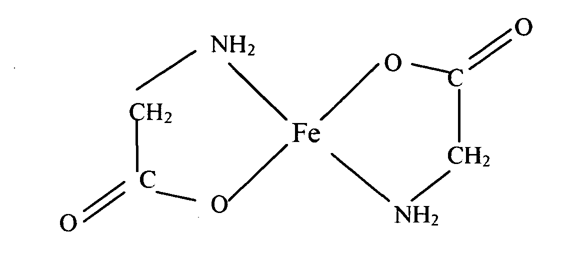

This compound, and its structure, have been known for some time (Ashmed S.D. The chemistry of ferrous bis-glycinate chelate, Arch. Latino Am. De Nutr., 2001, 51(1), 7-12*;* Atkins P.W., Berau J.A. 1992 General Chemistry 2nd ed. Scientific American Books, WH Freeman New York; Coplin et al. Tolerability of Iron: a comparison of bis-glycino iron II and ferrous sulphate, Clinical Therapeutics, vol. 13, n. 5, 606-612 , 1991*).*

Ferrous bisglycinate chelate is a quite soluble compound characterised by a metallic centre, Fe (II), tetracoordinated by two identical chealators (glycine). The dative bond of the nitrogen atom contributes to stabilising the energy of the electron orbitals of the metallic centre and the geometry of the binder, through the formation of a five-atom ring.

This compound is also known for its tolerability, safety and high bioavailability (Jeppsen R. B. Toxicology and safety of Ferrochel and other iron amino acids chelates, Arch. Latino Am. de Nutr., 2001, 51 (1), 26-34*;* Opinion if the scientific Panel on Food additives, Flavouring Processing Aids and materials in contact with Food on a request from the Commision related to: Ferrous bisglycinate as a source of iron for use in the manufacturing of foods and in foods supplements, EFSA Journal, 2006, 299, 1-17*).*

The literature highlights that such a chelate complex has greater bioavailability than iron mineral salts, like for example ferrous sulphate, since it is absorbed as such at the intestinal mucous and therefore does not undergo any chemical change in the gastrointestinal tract (Pineda O. et al., Effectiveness of iron amino acids chelate on the treatment of iron deficiency anaemia in adolescents, Journal of appl. Nutr., Vol. 46, Numbers 1-2, 1994*;* Pineda O., Effectiveness of treatment of iron deficiency anaemia in infants and young children with ferrous bis-glycinate chelate, Nutrition, 17, 2001, 381-84*).*

The stability of the bind, demonstrated by the fact that the product does not hydrolize at different pH values of the gastrointestinal tract, and the low molecular weight (204 g/mol) allow the maximum absorption when administered orally (DeWayne H.A., The absorbtion and metabolism of iron amino acid chelate, Arch. Latino Am. de Nutr., 2001, 51(1), 7-12; Marchetti M et al., Comparison of the rates of vitaminic degradation when mixed with metal sulphates or metal amino acids chelates, J. Food Comp. Anal., 200, 13, 875-884).

Such a compound has its most common application as food additive, above all in foods like milk and dairy products that do not contain iron.

Such a compound does, however, have a rather unpleasant taste, distinctive of the iron complex contained in it and, more generally, of all products containing iron that, notoriously, are thus difficult to administer.

Now it has surprisingly been found that the addition to the ferrous bisglycinate chelate of a particular mixture of sweeteners and flavourings in a certain ratio allows a formulation to be obtained with improved palatability, eliminating the unpleasant taste and thus making it easier to administer the compound.

In particular, said mixture consists of acesulfame K, sucralose, sorbitol and orange or lemon flavouring.

The object of the present invention is therefore formulations containing ferrous bisglycinate chelate in association with a mixture of acesulfame K, sucralose, sorbitol and flavouring, preferably orange or lemon, in which said mixture is in a ratio of about 1 : 0.40 : 0.18 : 3.20 by weight, respectively.

The formulations of the present invention can also contain excipients and/or physiologically acceptable additives like, for example, acidifiers and/or preservatives (ascorbic acid, methyl paraben).

The ferrous bisglycinate chelate is present in the formulations of the present invention in an amount that varies preferably from 60 to 400 mg/dose. More preferably, the ferrous bisglycinate chelate is present in an amount equal to 70 mg/dose or 210 mg/dose.

The pH generated by the formulations of the invention dissolved in water is preferably above 4, and more preferably between 4.5 and 7.5.

The aforementioned pH is thus generated by dissolving a formulation having a ferrous bisglycinate chelate content of between 60 and 400 mg in 100-150 ml of water.

The formulations of the present invention are preferably intended for oral administration and can more preferably be in the form of a tablet, soft or hard capsule, oral solution, oral suspension, granules, effervescent granules or dissolve-in-the-mouth powder.

Even more preferably, said tablet can be a dissolve-in-the-mouth, chewable, effervescent or swallowable tablet.

A further object of the present invention is the use of a formulation containing ferrous bisglycinate chelate in association with a mixture of acesulfame K, sucralose, sorbitol and flavouring, preferably powdered orange or lemon, in a ratio of 1 : 0.40 : 0.18 : 3.20 by weight, respectively, for the prevention and/or treatment of pathologies due to iron deficiency.

The following examples are intended to make it easier to understand the present invention without limiting it in any way.

### EXAMPLES

### Example 1

Formulation of an effervescent tablet:

| *Active ingredient:* | | *mg*/*tablet* |
|---|---|---|
| Ferrous bisglycinate chelate: 70 mg (corresponding to 14 mg of iron) | | |
| *Sweeteners:* | | |
| | Acesulfame K: | 25 mg |
| | Sucralose: | 10 mg |
| | Sorbitol: | 4.5 mg |
| *Flavouring* | | |
| Orange flavouring: | | 80 mg |

The acesulfame K:sucralose:sorbitol:flavouring ratio of the effervescent tablet of example 1 is 1:0.40:0.18:3.20.

The pH of the solution obtained by dissolving the effervescent tablet of example 1 in 150 ml of water is 4.7.

### Example 2

Formulation of a swallowable tablet

| *Active ingredient:* | *mg*/*tablet* |
|---|---|
| Ferrous bisglycinate chelate: 70 mg (corresponding to 14 mg of iron) | |
| Sweeteners: | |
| Acesulfame K: | 10 mg |
| Sucralose: | 4 mg |
| Sorbitol: | 1.8 mg |
| Flavouring | |
| Powdered lemon flavouring: | 32 mg |

The acesulfame K:sucralose:sorbitol:flavouring ratio of the tablet of example 2 is 1:0.40:0.18:3.20.

The pH of the solution obtained by dissolving the tablet of example 2 in 50 ml of water is 7.0.

### Example 3

Formulation of effervescent granules

| *Active ingredient:* | *mg*/*tablet* |
|---|---|
| Ferrous bisglycinate chelate: 210 mg (corresponding to 42 mg of iron) | |
| *Sweeteners:* | |
| Acesulfame K: | 25 mg |
| Sucralose: | 10 mg |
| Sorbitol: | 4.5 mg |
| Flavouring | |
| Orange flavouring : | 80 mg |

The acesulfame K:sucralose:sorbitol:flavouring ratio of the effervescent granules of example 3 is 1:0.40:0.18:3.20.

The pH of the solution obtained by dissolving the effervescent granules of example 3 in 150 ml of water is 4.7.

### Example 4

Formulation of solution/oral suspension

| *Active ingredient:* | *mg*/*tablet* |
|---|---|
| Ferrous bisglycinate chelate: 210 mg (corresponding to 42 mg of iron) | |
| *Sweeteners:* | |
| Acesulfame K: | 25 mg |
| Sucralose: | 10 mg |
| Sorbitol: | 4.5 mg |
| Flavouring | |
| Orange flavouring | 80 mg |

The acesulfame K:sucralose:sorbitol:flavouring ratio of the oral suspension of example 4 is 1:0.40:0.18:3.20.

The pH of the oral suspension solution (as such) is 5.5.

### EXPERIMENTAL PART

Three different preparations of effervescent granules were prepared, each containing the same dose of active ingredient (70 mg of ferrous bisglycinate) and different amounts of flavourings and sweeteners. The three preparations summarised in the table below. Preparation number 2 is the one with sweeteners and flavourings in the optimal ratios determined by us (and it is the one claimed by us).

| COMPONENT | PREPARATION 1 | PREPARATION 2 | PREPARATION 3 |
|---|---|---|---|
| Acesulfame K: | 15 mg | 25 mg | 35 mg |
| Sucralose: | 5 mg | 10 mg | 15 mg |
| Sorbitol: | 2 mg | 4.5 mg | 8 mg |

The three preparations underwent tasting by three different people, who were asked to judge their palatability, expressed in numerical form, according to the table below.

| JUDGEMENT | PALATABILITY |
|---|---|
| 1 | Unacceptable |
| 2 | Fair |
| 3 | Good |
| 4 | Excellent |

The judgements expressed by the three subjects are summarised in the following table, together with the calculated average values

| SUBJECT | PREPARATION 1 | PREPARATION 2 | PREPARATION 3 |
|---|---|---|---|
| A | 1 | 3 | 2 |
| B | 2 | 4 | 2 |
| C | 2 | 4 | 2 |
| AVERAGE | 1.7 | 3.7 | 2 |

The results indicate that the best formulation is the one of preparation 2.

## Claims

1. Pharmaceutical formulation containing ferrous bisglycinate chelate in association with a mixture of acesulfame K, sucralose, sorbitol and flavouring wherein the compounds of said mixture are in a ratio of about 1 : 0.40 : 0.18 : 3.20 by weight, respectively.

2. Formulation according to claim 1 wherein the ferrous bisglycinate chelate is present in an amount that varies from 60 to 400 mg/dose.

3. Formulation according to claim 2 wherein the ferrous bisglycinate chelate is present in an amount equal to 70 mg/dose.

4. Formulation according to claim 2 wherein the ferrous bisglycinate chelate is present in an amount equal to 210 mg/dose.

5. Formulation according to claim 1 wherein a dose of said formulation containing from 60 to 400 mg of ferrous bisglycinate chelate dissolved in 100-150 ml of water generates a pH of over 4.

6. Formulation according to claim 5 wherein said pH is between 4.5 and 7.5.

7. Formulation according to claim 1 wherein said flavouring is preferably selected from powdered orange and lemon flavouring.

8. Formulation according to one of the previous claims in the form of a tablet, soft or hard capsule, oral solution, oral suspension, granules, effervescent granules or dissolve-in-the-mouth powder.

9. Formulation according to claim 8 wherein said tablet is a dissolve-in-the-mouth, chewable, effervescent or swallowable tablet.

10. Formulation according to one of the previous claims also containing excipients and/or physiologically acceptable additives.

11. Use of the formulation according to claims 1-10 for the prevention and/or treatment of pathologies due to iron deficiency.

## Patentansprüche

1. Pharmazeutische Formulierung, enthaltend Eisen(II)bisglycinatchelat zusammen mit einer Mischung aus Acesulfam K, Sucralose, Sorbit und Geschmacksstoffen, wobei die Verbindungen der Mischung in einem Verhältnis von etwa 1:0,40:0,18:3,20 (Gew.) vorliegen.

2. Formulierung gemäss Anspruch 1, wobei das Eisen(II)bisglycinatchelat in einer Menge vorliegt, die von 60 bis 400 mg/Dosis variiert.

3. Formulierung gemäss Anspruch 2, wobei das Eisen(II)bisglycinatchelat in einer Menge gleich 70 mg/Dosis vorliegt.

4. Formulierung gemäss Anspruch 2, wobei das Eisen(II)bisglycinatchelat in einer Menge gleich 210 mg/Dosis vorliegt.

5. Formulierung gemäss Anspruch 1, wobei eine Dosis der Formulierung, die 60 bis 400 mg Eisen(II)bisglycinatchelat, gelöst in 100 bis 150 ml Wasser, enthält, einen pH-Wert von über 4 erzeugt.

6. Formulierung gemäss Anspruch 5, wobei der pH-Wert zwischen 4,5 und 7,5 beträgt.

7. Formulierung gemäss Anspruch 1, wobei der Geschmacksstoff vorzugsweise aus pulverisiertem Orangen- und Zitronenaroma ausgewählt ist.

8. Formulierung gemäss einem der vorhergehenden Ansprüche in Form einer Tablette, einer Weich- oder Hartkapsel, einer oralen Suspension, Granulaten, Brausegranulaten oder einem sich im Mund auflösenden Pulver.

9. Formulierung gemäss Anspruch 8, wobei die Tablette eine sich im Mund auflösende, kaubare, Brause- oder schluckbare Tablette ist.

10. Formulierung gemäss einem der vorhergehenden Ansprüche, die auch Hilfsstoffe und/oder physiologisch annehmbare Additive enthält.

11. Verwendung der Formulierung gemäss Ansprüchen 1 bis 10 zur Prävention und/oder Behandlung von Krankheiten aufgrund von Eisenmangel.

## Revendications

1. Formulation pharmaceutique contenant un chélate de bisglycinate ferreux en association avec un mélange d'acésulfame K, de sucralose, de sorbitol et d'agent aromatisant dans laquelle les composés dudit mélange sont dans un rapport d'environ 1/0,40/0,18/3,20 en poids, respectivement.

2. Formulation selon la revendication 1 dans laquelle le chélate de bisglycinate ferreux est présent en une quantité qui varie de 60 à 400 mg/dose.

3. Formulation selon la revendication 2 dans laquelle le chélate de bisglycinate ferreux est présent en une quantité égale à 70 mg/dose.

4. Formulation selon la revendication 2 dans laquelle le chélate de bisglycinate ferreux est présent en une quantité égale à 210 mg/dose.

5. Formulation selon la revendication 1 dans laquelle une dose de ladite formulation contenant de 60 à 400 mg de chélate de bisglycinate ferreux dissous dans 100 à 150 ml d'eau génère un pH supérieur à 4.

6. Formulation selon la revendication 5 dans laquelle ledit pH est compris entre 4,5 et 7,5.

7. Formulation selon la revendication 1 dans laquelle ledit agent aromatisant est de préférence choisi parmi un agent aromatisant en poudre à base d'orange et de citron.

8. Formulation selon l'une quelconque des revendications précédentes sous la forme d'un comprimé, d'une capsule molle ou dure, d'une solution orale, d'une suspension orale, de granules, de granulés effervescents ou d'une poudre à dissoudre dans la bouche.

9. Formulation selon la revendication 8 dans laquelle ledit comprimé est un comprimé à dissoudre dans la bouche, à mâcher, effervescent ou à avaler.

10. Formulation selon l'une quelconque des revendications précédentes contenant également des excipients et/ou des additifs physiologiquement acceptables.

11. Utilisation de la formulation selon les revendications 1 à 10 pour la prévention et/ou le traitement de pathologies dues à une carence en fer.
